Europäisches Patentamt

European Patent Office

Office européen des brevets

(11) Publication number: **0 212 332**
**B1**

(12) EUROPEAN PATENT SPECIFICATION

(45) Date of publication of patent specification: **25.04.90**

(21) Application number: **86110256.4**

(22) Date of filing: **09.12.82**

(60) Publication number of the earlier application in accordance with Art. 76 EPC: **0 081 438**

(51) Int. Cl.⁵: **A 61 F 13/02, A 61 L 15/16**

(54) Adhesive bandage.

(30) Priority: **09.12.81 JP 198879/81**
**14.12.81 JP 186996/81**

(43) Date of publication of application:
**04.03.87 Bulletin 87/10**

(45) Publication of the grant of the patent:
**25.04.90 Bulletin 90/17**

(84) Designated Contracting States:
**AT BE CH DE FR GB IT LI LU NL SE**

(56) References cited:
**US-A-2 489 675**
**US-A-2 579 403**
**US-A-2 595 606**
**US-A-2 714 382**
**US-A-3 297 032**
**US-A-3 366 112**
**US-A-3 580 254**
**US-A-4 162 672**
**US-A-4 192 299**

(73) Proprietor: **NIPPON ZOKI PHARMACEUTICAL CO. LTD.**
**10, 2-chome Hirano-machi Higashi-ku**
**Osaka-shi Osaka-fu (JP)**

(72) Inventor: **Konishi, Ryusaku**
**Sakasegawa Manshion A 707 1-3, Sakasedai**
**Takarazuka-shi Hyogo-ken (JP)**

(74) Representative: **Tony-Durand, Serge**
**Cabinet Tony-Durand 77, rue Boissière**
**F-75116 Paris (FR)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

1. Field of the invention
   The invention relates to an adhesive bandage.

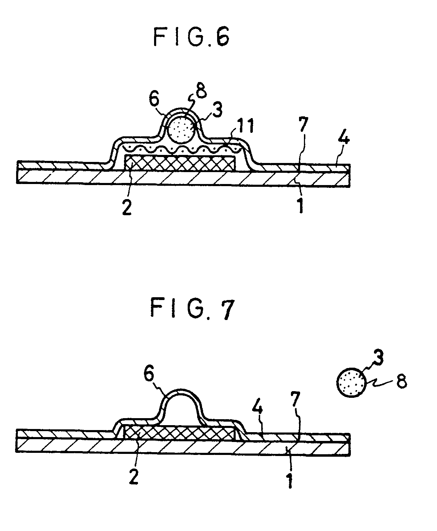

2. Description of the prior art:
   It is already known by US—A—3297032, an adhesive bandage comprising an adhesive tape, an absorbent pad carried by the tape and a release sheet, releasably attached to the tap on the side of the adhesive. In the embodiment of figure 6, provision is made for a capsule containing a medicament. This capsule, which can be ruptured by pressure, is placed between a pad carried by the adhesive tape of the bandage and the removable protective element. However, in this bandage, said removable element extends over only part of the length of the adhesive tape, namely over one of the portions located on one side of the absorbent pad as well as opposite to the pad itself. In fact another separate removable protective element is added on the other portion of the adhesive tape. The capsule containing the medicament is therefore placed between the free end portion of the first protective element and the absorbent pad. The edge of this end portion is completely free with respect to the absorbent pad as well as with respect to the other elements of the bandage. Although this is not specified in the U.S. patent aforesaid, it may be supposed that the capsule containing the medicament adheres either to the outer face of the absorbent pad or the inner face of the first removable protective element. However, even if this is the case, this capsule is very badly protected against the risks of degradation before the corresponding bandage is employed. This capsule is therefore liable to be torn away accidentally during handling. Moreover, the fact that the edge of the first protective element which covers the absorbent pad remains free and unattached has a disadvantage in that said absorbent pad is in no way protected against the dangers of soiling prior to use of the bandage. In consequence, the absorbent pad cannot remain sterile up to the moment of use. This therefore results in serious dangers of infection.
   US—A—3580254 and US—A—2714382 illustrate other bandages of that kind, comprising an adhesive tape and a absorbent pad or the like with a releaseable protecting element.

Summary of the invention
   The object of the invention is to make a certain number of improvements to the bandages in which provision is made for a rupturable capsule containing a medicament which is intended to impregnate a pad carried by the adhesive tape.
   Thus the primary object of the invention is to ensure perfect protection of said capsule prior to use of the bandage by ensuring that said capsule is completely enclosed and that it is not liable to suffer degradation prior to use.
   However, the invention also has for its object to ensure protection of the aforesaid pad in order to guard against any danger of soiling or contamination of this latter prior to use of the bandage.
   According to the invention, the adhesive bandage comprises an adhesive tape, an absorbent pad carried by the tape and a release sheet, releasably attached to the tape on the side of the adhesive, wherein said absorbent pad is smaller in its length that the tape, said bandage further comprising a medicine encapsulated into a capsule rupturable under pressure and which is placed between said absorbent pad and said release sheet, said adhesive bandage being characterized in that:
   —said release sheet is overlaying the whole tape,
   —and said release sheet comprises a blister portion having an open side facing the pad, and in which is placed said capsule containing the medicine.
   Other objects and features of the present invention will be more apparent to those skilled in the art on consideration of the accompanying drawings and following specification wherein are disclosed several exemplary embodiments of the invention with the understanding that such variations, modifications and elimination of parts may be made therein as fall within the scope of the appended claims without departing from the spirit of the invention.
   Figure 1 is a top plan view of an adhesive bandage according to the invention;
   Figure 2 is a sectional view of the embodiment taken along the line A—A' of Figure 1;
   Figure 3 is a cross sectional view of a capsule in the adhesive bandage of this invention;
   Figures 4 and 5 are sectional views of blisters according to further embodiments of this invention; and
   Figures 6 and 7 are sectional views of further embodiments of this invention.

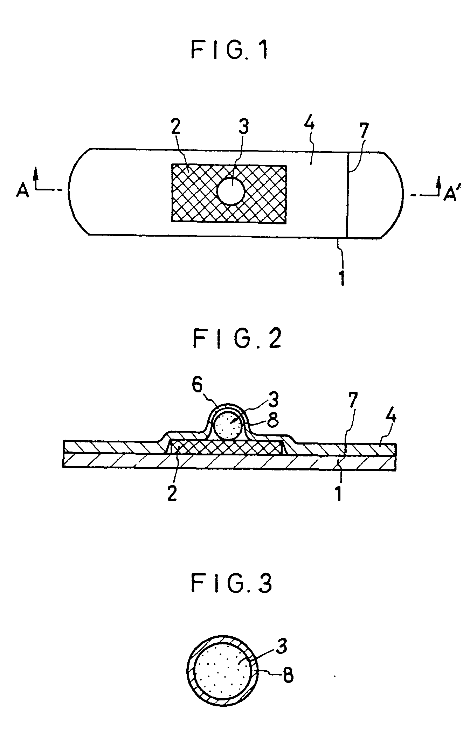

Detailed description of the invention
   The adhesive bandage of this invention comprises an adhesive tape carrying a pad thereon, and a release sheet holding a medicine separately from the pad.
   The invention will be described in more detail by way of example with reference to the drawings. Referring first to Figures 1 and 2, an adhesive bandage embodying this invention comprises an adhesive tape 1 carrying a pad 2 thereon, and a medicine 3 enclosed in a capsule 8 as hereinafter described.
   The release sheet 4 may be provided at one end with a slit 7 along which the release sheet 4 may be bent so that is may be easily removed. An appropriate quantity of the medicine is put into the capsule 8, itself placed in a blister (recess) formed at a predetermined position of the release sheet 4.
   The adhesive bandage of this invention may contain various kinds of medicines, for example, a sterilizer or disinfectant, a wound astringent healing promoter, a hemostatic agent, an anti-inflammatory agent, an antihistaminen, and a local anesthetic. Specific examples of the sterilizer or disinfectant include chlorhexidine gluconate, benzalkonium chloride, chloroxylénol, acrinol, thianthol, dequalinium chloride, sulfisomidine, sulfamine, nitrofurazone, boric acid, homosulfamine, and triclocarban. Examples of the healing promoter include zinc oxide, pyridoxine hydrochloride, tocopherol acetate, and pyridoxine dipalmintate. Examples of the hemostatic agent include naphazoline hydrochloride, zinc sulfate, and ephedrine hydrochloride. Examples of the anti-inflammatory agent include steroids such as prednisolone, dexamethasone, cortisone acetate and other steroids, glycyrrhetinic acid, and lysozyme chloride. Examples of the atihistamine include chlorophenilamine maleate and diphenhydramine hydrochloride. Examples of the local anesthetic include lidocaine, ethyl aminobenzoate, procaine hydrochloride, dibucaine hydrochloride, tetracaine hydrochloride, and diethyl aminoethyl p-butylaminobenzoate hydrochloride.

These medicines may be used singly or in combination depending upon intended purposes. They may be in the form of a solution, ointments, grease or powder, or any other form as long as they are fluid. If appropriate, a solution may be absorbed into carrier such as a sponge or cotton and thereafter enclosed in the blister.

The adhesive tape may be of any ordinary material and constructions and preferably it is porous. If required, the adhesive tape may have a recessed center to place the pad, and a waterproof rear surface which is obtained by any customary method.

The pad may be formed from absorbent cotton or any other type of cotton, an unwoven fabric, paper, sponge, or any other liquid-absorbent material. When a large bandage is required, a plurality of blisters may be provided for one pad.

The capsule 8 (Fig. 3) is formed from gelatin, or any other pharmaceutically acceptable film forming material that does not react with the medicine. The form of the capsule may be selected depending on the desired purpose and it may be, for example, sphere, cylinder, cone or square column. In order to be placed in a gelatin capsule, the medicine is usually dissolved or suspended in a vegetable oil, such as sesame, peanut or olive oil. The medicine does not necessarily need to be in the form of a solution, but may be in the form of an ointment, grease or powder, or any other form if it is free flowing. The use of a solution is, however, most desirable when the medicine is placed in the capsule.

When the bandage is to be applied to a wound, a finger pressure is applied to the blister 6 to rupture the capsule 8 to cause the medicine to flow from the capsule to the pad. Then, the release sheet 4 is bent along its slit 7, and removed from the adhesive tape 1, whereby the pad 2 holding a fresh supply of medicine is exposed for application to the wound. The same advantages as described above can be achieved by the bandage of this type. The medicine does not lose its effect during the storage of the bandage, since it is enclosed in the capsule immediately before the use of the bandage. The pad can be applied to the wound without hurting it and give rise to a pain, since it is wetted with the medicine immediately prior to its application. The medicine can maintain its efficacy for a prolonged period for achieving any desired sterilizing, disinfecting and curing purposes. And also the bandage permits the visual observation of the medicine if the capsule and the blister portion are transparent.

The bandage may have a plurality of capsules depending on the sizes of the pad and the bandage.

When the capsule 8 is ruptured, its fragments adhere closely to the inner wall surface of the blister, and do not remain on the pad, if the blister is provided with an irregular or uneven inner surface. Even if any fragment of the capsule material may be left on the pad, it does not give rise to any problem if the capsule is formed pharmaceutically acceptable material such as gelatin. If desired an adhesive may be applied on the inner surface of the blister so as to remove the ruptured capsule attaching to the release sheet.

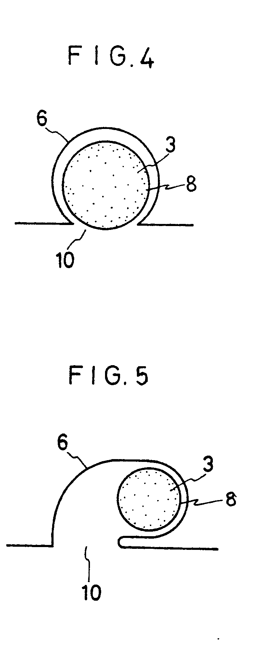

Modified forms of the blister are shown in Figures 4 and 5. Figure 4 shows a blister having the shape of a superior arc, while the blister shown in Figure 5 has the shape of a modified superior arc. Due to said form, the blister portion 6 is bigger in width than the capsule, in order to have a free space in said blister. These modified blisters are effective for presenting any capsule material from falling on the pad after rupture of the capsule. The blister shown in Figure 4 has an opening 10 which is smaller than the diameter of the capsule, but this feature is not required as for the blister having a modified superior arc profile as shown in Figure 5. Further, as shown in Figure 6, a screen 11 or the like may be introduced between the capsule 8 and the pad 2 to facilitate the puncture of the capsule and the removal of the capsule fragments. The screen may be wire mesh, plastic mesh, cloth etc.

Although the above explanation is made on the embodiments in which the capsule is incorporated into the blister portion in manufacturing the bandage, the invention can be also applied to such adhesive bandage that an encapsulated medicine is stored in the state that it is disengaged from the blister portion and that it is incorporated into the blister portion when use. That is, as shown in Figure 7, the encapsulated medicine is detached from the blister portion during storage, and when the bandage is applied to a wound, the encapsulated medicine is inserted into the blister portion.

**Claims**

1. An adhesive bandage, comprising an adhesive tape (1), an absorbent pad (2) carried by the tape and a release sheet (4), releaseably attached to the tape on the side of the adhesive, wherein said absorbent pad (2) is smaller in its length that the tape (1), said bandage further comprising a medicine encapsulated into a capsule (8) rupturable under pressure and which is placed between said absorbent pad (2) and said release sheet (4), characterized in that:

—said release sheet (4) is overlaying the whole tape (1),

—and said release sheet (4) comprises a blister portion (6) having an open side facing the pad (2), and in which is placed said capsule (8) containing the medicine (2).

2. An adhesive bandage as claimed in Claim 1, characterized in that the capsule is made from gelatin.

3. An adhesive bandage as claimed in Claim 1, characterized in that the medicine is dissolved into or suspended oil selected from the group substantially consisting of sesame, peanut and olive oil.

4. An adhesive bandage as claimed in Claim 1, characterized in that the blister portion is designed in a form of superior arc.

5. An adhesive bandage as claimed in Claim 4, characterized in that the blister portion (6) is bigger in width that the capsule (8), in order to have a free space in said blister.

6. An adhesive bandage as claimed in Claim 1, characterized in that the capsule is designed in a form of sphere, cylinder, cone or square column.

7. An adhesive bandage as claimed in Claim 1, characterized in that a screen is interposed between the pad and the capsule.

8. An adhesive bandage as claimed in Claim 1, characterized in that the medicine is selected from the group substantially consisting of sterilizer, disinfectant, wound astringent healing promoter, hemostatic agent, anti-inflammatory agent, antihistamine and local anesthetic.

9. An adhesive bandage as claimed in Claim 1, characterized in that the release sheet is provided with a slit at one end thereof.

10. An adhesive bandage as claimed in Claim 1, characterized in that the medicine is in a form of solution.

11. An adhesive bandage as claimed in Claim 1, characterized in that the medicine is in a form of ointment.

12. An adhesive bandage as claimed in Claim 1, characterized in that the medicine is in a form of grease.

13. An adhesive bandage as claimed in Claim 1, characterized in that the medicine is in a form of powder.

14. An adhesive bandage as claimed in Claim 1, characterized in that the pad is made from a material selected from the group substantially consisting of absorbent cotton, unwoven fabric, paper and sponge.

15. An adhesive bandage as claimed in Claim 1, characterized in that the blister portion is designed in a form selected from the group substantially consisting of spere, ellipse, and rectangle.

16. An adhesive bandage as claimed in Claim 1, characterized in that plurality of blisters facing a single pad.

17. An adhesive bandage as claimed in Claim 1, characterized in that the blister portion and/or the capsule is transparent.

**Patentansprüche**

1. Heftverband mit einem Klebestreifen (1), einem von Streifen getragenen absorbierenden Polster (2) und einer abziehbaren Schicht (4), die auf der Klebstoffseite des Streifens lösbar befestigt ist, wobei der absorbierende Polster (2) eine geringere Länge hat als der Streifen (1), und wobei der genannte Verband weiters ein Medikament umfaßt, welches in einer Kapsel (8) eingeschlossen ist, die unter Druck zerreißbar und zwischen dem absorbierenden Polster (2) und der abziehbaren Schicht (4) angeordnet ist, dadurch gekennzeichnet, daß die abziehbare Schicht (4) sich über den gesamten Streifen (1) erstreckt, und die abziehbare Schicht (4) einen blasenartigen Abschnitt (6) umfaßt, der eine zum Polster (2) gerichtete offene Seite aufweist und in dem die das Medikament (3) enthaltende Kapsel (8) angeordnet ist.

2. Heftverband nach Anspruch 1, dadurch gekennzeichnet, daß die Kapsel aus Gelatine besteht.

3. Heftverband nach Anspruch 1, dadurch gekennzeichnet, daß das Medikament in öl, ausgewählt aus der im wesentlichen aus Sesam-, Erdnuß- und Olivenöl bestehenden Gruppe, gelöst oder suspendiert ist.

4. Heftverband nach Anspruch 1, dadurch gekennzeichnet, daß der blasenförmige Abschnitt die Form eines nach oben gewölbten Bogens aufweist.

5. Heftverband nach Anspruch 4, dadurch gekennzeichnet, daß der blasenförmige Abschnitt (6) breiter ist als die Kapsel (8), um in der genannten Blasen einen Freiraum zu haben.

6. Heftverband nach Anspruch 1, dadurch gekennzeichnet, daß die Kapsel kugelförmig, zylinderförmig, kegelförmig oder quaderförmig ist.

7. Heftverband nach Anspruch 1, dadurch gekennzeichnet, daß zwischen dem Polster und der Kapsel ein Gitter angeordnet ist.

8. Heftverband nach Anspruch 1, dadurch gekennzeichnet, daß das Medikament ausgewählt wird aus der Gruppe, die im wesentlichen Sterilisiermittel, Desinfektionsmittel, adstringierende heilungsbeschleunigende Mittel, Hämostypica, entzündungshemmende Mittel, Antihistaminika und Lokalanästhetika umfaßt.

9. Heftverband nach Anspruch 1, dadurch gekennzeichnet, daß die abziehbare Schicht an einem Ende mit einem Schlitz versehen ist.

10. Heftverband nach Anspruch 1, dadurch gekennzeichnet, daß das Medikament eine Lösung ist.

11. Heftverband nach Anspruch 1, dadurch gekennzeichnet, daß das Medikament eine Salbe ist.

12. Heftverband nach Anspruch 1, dadurch gekennzeichnet, daß das Medikament schmierenartig ist.

13. Heftverband nach Anspruch 1, dadurch gekennzeichnet, daß das Medikament puderförmig ist.

14. Heftverband nach Anspruch 1, dadurch gekennzeichnet, daß der Polster aus einem Material, ausgewählt aus der im wesentlichen absorbierende Baumwolle, unverwobenen Stoff, Papier und Schwamm umfassenden Gruppe, hergestellt ist.

15. Heftverband nach Anspruch 1, dadurch gekennzeichnet, daß der blasenartige Abschnitt eine Form aufweist, die ausgewählt wird aus der im wesentlichen aus Kugel, Ellipse und Rechteck bestehenden Gruppe.

16. Heftverband nach Anspruch 1, dadurch gekennzeichnet, daß mehrere, einem einzigen Polster gegenüberliegende Blasen vorgesehen sind.

17. Heftverband nach Anspruch 1, dadurch gekennzeichnet, daß der blasenartige Abschnitt und/oder die Kapsel durchsichtig sind.

## Revendications

1. Emplâtre adhésif, comprenant un ruban adhésif (1), un tampon absorbant (2) porté par le ruban et une feuille de dégagement (4) attachée de manière amovible sur la face adhésive du ruban, dans lequel ledit tampon absorbant (2) est plus court que le ruban (1), ledit emplâtre comprenant en outre un médicament (3) encapsulé dans une capsule (8) pouvant être rompue par pression et qui est placée entre ledit tampon absorbant (2) et ladite feuille de dégagement (4), caractérisé en ce que:
—ladite feuille de dégagement (4) recouvre tout le ruban (1),
—et ladite feuille de dégagement (4) comprend une partie formant une bulle (6) ayant une face ouverte faisant face au tampon (2), et dans laquelle est placée ladite capsule (8) contenant le médicament (3).

2. Emplâtre adhésif selon la revendication 1, caractérisé en ce que la capsule est faite en gélatine.

3. Emplâtre adhésif selon la revendication 1, caractérisé en ce que le médicament est dissous ou mis en suspension dans une huile choisie dans le groupe se composant essentiellement des huiles de sésame, d'arachide et d'olive.

4. Emplâtre adhésif selon la revendication 1, caractérisé en ce que la partie formant une bulle est conçue sous la forme d'un arc supérieur.

5. Emplâtre adhésif selon la revendication 4, caractérisé en ce que la partie formant une bulle (6) est plus large que la capsule (8) afin d'avoir un espace libre dans ladite bulle.

6. Emplâtre adhésif selon la revendication 1, caractérisé en ce que la capsule est conçue sous la forme d'une sphère, d'un cylindre, d'un cône colonne carrée.

7. Emplâtre adhésif selon la revendication 1, caractérisé en ce qu'un écran est interposé entre le tampon et la capsule.

8. Emplâtre adhésif selon la revendication 1, caractérisé en ce que le médicament est choisi dans le groupe se composant essentiellement d'agents stérilisants, désinfectants, promoteurs astringents de cicatrisation, agents hémostatiques, anti-inflammatoires, anti-histaminiques et anesthésiques locaux.

9. Emplâtre adhésif selon la revendication 1, caractérisé en ce que la feuille de dégagement est munie d'une fente à une de ses extrémités.

10. Emplâtre adhésif selon la revendication 1, caractérisé en ce que le médicament est sous la forme d'une solution.

11. Emplâtre adhésif selon la revendication 1, caractérisé en ce que le médicament est sous une forme d'onguent.

12. Emplâtre adhésif selon la revendication 1, caractérisé en ce que le médicament est sous une forme de graisse.

13. Emplâtre adhésif selon la revendication 1, caractérisé en ce que le médicament est sous une forme de poudre.

14. Emplâtre adhésif selon la revendication 1, caractérisé en ce que le tampon est fait d'une matière choisie dans le groupe se composant essentiellement de coton absorbant, de produit non-tissé, de papier de d'éponge.

15. Emplâtre adhésif selon la revendication 1, caractérisé en ce que la partie en forme de bulle est conçue dans une forme choisie dans le groupe se composant essentiellement de sphère, ellipse et rectangle.

16. Emplâtre adhésif selon la revendication 1, caractérisé en ce qu'il comporte une pluralité de bulles faisant face à un seul tampon.

17. Emplâtre adhésif selon la revendication 1, caractérisé en ce que la partie en forme de bulle et/ou la capsule est transparente.

FIG.1

FIG.2

FIG.3

FIG.4

FIG.5

FIG.6

FIG.7